# EUROPÄISCHE PATENTSCHRIFT

(11) **EP 2 200 760 B1**
(45) Veröffentlichungstag und Bekanntmachung des Hinweises auf die Patenterteilung: **07.03.2012**
(21) Anmeldenummer: 08802678.6
(22) Anmeldetag: 26.09.2008
(51) Int. Cl.: B08B 3/08

(54) **Vorrichtung und Verfahren zur Reinigung geschlossener Räume**
Device and method for cleaning closed spaces
Dispositif et procédé pour nettoyer des espaces fermés

(30) Priorität: 27.09.2007 DE 202007013556 U
(43) Veröffentlichungstag der Anmeldung: 30.06.2010
(73) Patentinhaber: Koch, Peter, 7423 Pinkafeld (AT)
(72) Erfinder: Koch, Peter, 7423 Pinkafeld (AT)
(74) Vertreter: Hagemann, Heinrich
(86) Internationale Anmeldenummer: PCT/EP2008/008234
(87) Internationale Veröffentlichungsnummer: WO 2009/043559

(56) Entgegenhaltungen:
- US-A1- 2003 140 945
- US-A1- 2004 221 877
- US-A1- 2005 133 067

## Beschreibung

Die Erfindung betrifft eine Vorrichtung zur Reinigung geschlossener Räume mit einem Dampferzeuger und/oder Befeuchter und einer Ventilationseinrichtung, die mit einer Steuereinrichtung derart gekoppelt sind, dass die Zeit der Dampfeinleitung in den geschlossenen Raum und die Einwirkzeit des Dampfes im geschlossenen Raum steuerbar sind. Die Erfindung betrifft ferner ein Verfahren zum Reinigen geschlossener Räume.

DE 26 53 548 offenbart eine Vorrichtung zur Desinfektion geschlossener Räume mit Hilfe von Formalin, bei dem das Formalin in wässriger Lösung verdampft und der Dampf der Raumatmosphäre zugesetzt wird. Die bekannte Vorrichtung umfasst einen Behälter für das flüssige Formalin, wobei unter dem Behälter eine Heizplatte angeordnet ist, die dazu dient, den Behälter zu erwärmen, so dass das Formalin in den gasförmigen Zustand überführt wird. Das verdampfte Formalin wird in den geschlossenen Raum eingeleitet, wobei die Zeiträume für die Verdampfung und die Einwirkung durch eine im Gerät befindliche Steuereinrichtung gesteuert werden. Ferner weist die bekannte Vorrichtung einen zweiten Behälter auf, in dem ein Ventilator angeordnet ist. Durch Lüftungsschlitze im unteren Bereich des zweiten Behälters wird nach der Formalinbedampfung des Raumes die Raumluft mit Hilfe des Ventilators durch die Lüftungsschlitze angesaugt und durch einen Filter wieder in den Raum geleitet, so dass das Formalin der Raumluft entzogen wird.

Die bekannte Vorrichtung hat den Nachteil, dass das Formalin selbst nach der Luftreinigung nicht vollständig abgebaut wird, so dass weiterhin Rückstände des Formalins in Form von Formaldehyd im gereinigten Raum verbleiben. Es ist bekannt, dass Formaldehyd eine gesundheitsschädliche, insbesondere karzinogene, Wirkung hat. Ferner ist die Filterung der Luft, die zur Reduktion der Formaldehydkonzentration nach dem Reinigungsvorgang zwingend erforderlich ist, zeitaufwändig. Außerdem ist die zu reinigenden Raumgröße dadurch begrenzt, dass die Menge des in der Vorrichtung vorgehaltenen Formalins limitiert ist.

Weitere Vorrichtungen und entsprechenden Verfahren sind aus den Druckschriften US 2005/0133067, US 2004/0221877, US 2003/014945, US 2006/0263281 und US 3549528 bekannt.

Der Erfindung liegt daher die Aufgabe zu Grunde, eine Vorrichtung zur Reinigung geschlossener Räume anzugeben, die eine schnelle, kostengünstige und rückstandsarme Reinigung geschlossener Räume ermöglicht. Ferner liegt der Erfindung die Aufgabe zugrunde, ein Verfahren zum Reinigen geschlossener Räume anzugeben.

Erfindungsgemäß wird diese Aufgabe im Hinblick auf die Vorrichtung dadurch gelöst, dass ein Ozonisator vorgesehen ist, der mit der Ventilationseinrichtung und/oder der Steuereinrichtung derart gekoppelt ist, dass die Zeit der Ozoneinleitung in den geschlossenen Raum und/oder die Einwirkzeit des Ozons im geschlossenen Raum steuerbar ist. Im Hinblick auf das Verfahren wird die Aufgabe durch den Gegenstand des Anspruchs 11 gelöst.

Der Erfindung liegt der Gedanke zu Grunde, eine Vorrichtung zur Reinigung geschlossener Räume mit einem Dampferzeuger und/oder Befeuchter und einer Ventilationseinrichtung anzugeben, die mit einer Steuereinrichtung gekoppelt sind derart, dass die Zeit der Dampfeinleitung und/oder der befeuchteten Luft in den geschlossenen Raum und die Einwirkzeit des Dampfes und/oder der befeuchteten Luft im geschlossenen Raum steuerbar sind, wobei ein Ozonisator vorgesehen ist, der mit der Ventilationseinrichtung und/oder der Steuereinrichtung derart gekoppelt ist, dass die Zeit der Ozoneinleitung in den geschlossenen Raum und/oder die Einwirkzeit des Ozons im geschlossenen Raum steuerbar ist.

Die erfindungsgemäße Vorrichtung hat den Vorteil, dass zur Reinigung von geschlossenen Räumen auf chemische Mittel verzichtet werden kann. Erfindungsgemäß wird statt dessen Ozon eingesetzt, das durch Aufbrechen von Molekülen eine Reinigungswirkung erzielt. Dabei bewirkt Ozon ebenfalls ein Aufbrechen von Geruchsmolekülen, so dass nicht nur mikrobiologische Verschmutzungen, sondern auch Gerüche eliminiert werden. Durch die Kombination mit Wasserdampf bzw. befeuchteter Luft wird ferner erreicht, dass das erzeugte Ozon rückstandsfrei zerstört wird. Vorteilhafterweise erfolgt der Reinigungsvorgang mit Hilfe der Steuereinrichtung automatisiert, so dass sich während der Raumreinigung keine Personen im Raum befinden müssen. Dadurch und durch die Zerstörung des Ozons durch den Wasserdampf wird gewährleistet, dass keine gesundheitlichen Schäden auftreten. Da das Ozon aus der Raumluft erzeugt wird, ist die Größe des zu reinigenden Raumes nicht durch die Reinigungsmittelmenge begrenzt, insbesondere wenn die Vorrichtung an eine Wasserversorgung angeschlossen ist. Die Einleitung von Wasserdampf in den Raum ermöglicht außerdem eine schnelle Zerstörung des Ozons, so dass der Reinigungsvorgang zügig abgeschlossen ist.

Der Befeuchter kann als Hochdruckbefeuchter oder Ultraschallbefeuchter ausgebildet sein. Andere Arten von Dampferzeugern bzw. Befeuchtern sind möglich.

Es ist möglich, dass die Ventilationseinrichtung mit der Steuereinrichtung gekoppelt ist, so dass die zugeführte Luftmenge, d.h. die Zuluftmenge, variierbar ist. Die Ventilationseinrichtung kann also eine veränderbare Zuluftmenge bzw. Frischluftzufuhr zulassen.

Die erfindungsgemäße Vorrichtung kann im Umluftbetrieb oder im Zuluftbetrieb verwendet werden. Durch die veränderbare Zuluftmenge ist es möglich, die Ozonkonzentration in dem zu reinigenden Raum zu beeinflussen, insbesondere bei Umluftbetrieb. Das Reinigungsergebnis wird dadurch verbessert bzw. die Effizienz des Reinigungsvorgangs erhöht.

Erfindungsgemäß ist die Steuereinrichtung derart angepasst, dass das Ozon und der Dampf und/oder die befeuchtete Luft wechselweise in den geschlossenen Raum einleitbar sind. Auf diese Weise kann die Einwirkzeit des Ozons gesteuert werden, so dass ein optimales Reinigungsergebnis ermöglicht wird. Die Einleitung von Dampf und/oder befeuchteter Luft ermöglicht es, eine für die Raumreinigung angepasste Luftfeuchte einzustellen. In besonders vorteilhafter Weise wird der Reinigungsvorgang mit der Dampfeinleitung bzw. der Einleitung befeuchteter Luft beendet, so dass die Ozonkonzentration im Raum durch den Wasserdampf minimiert wird.

Bei einer bevorzugten Ausführungsform der erfindungsgemäßen Vorrichtung ist die Steuereinrichtung mit einem Messwertaufnehmer, insbesondere zur Messung von Temperatur und/oder Luftfeuchte und/oder Ozonkonzentration, gekoppelt. Vorzugsweise ist die Steuereinrichtung derart angepasst, dass die Einleit- und/oder Einwirkzeiträume des Ozons und/oder des Dampfs in Abhängigkeit der Messwerte des Messwertaufnehmers steuerbar sind. Das hat den Vorteil, dass der Reinigungsvorgang individuell an die Bedingungen im jeweiligen geschlossenen Raum angepasst werden kann. Die Vorrichtung kann somit, zeit- und energiesparend betrieben werden.

Bei einer weiteren bevorzugten Ausführungsform der erfindungsgemäßen Vorrichtung ist dem Ozonisator ein Luftverdichter vorgeordnet. Durch die Zufuhr von verdichteter Luft wird die Effizienz der Ozonerzeugung erhöht. Es ist auch möglich, dass der Luftverdichter die Funktion eines Lufttrockners übernimmt, so dass dem Ozonisator getrocknete Luft zugeführt wird.

Dem Luftverdichter kann ein Sauerstoffkonzentrator nachgeordnet sein, der Sauerstoff aus der Umgebungsluft verdichtet und dem Ozonisator zuführt. Auf diese Weise kann die Effizienz der Ozonerzeugung weiter gesteigert werden, da der Anteil an Sauerstoffmolekülen zur Umwandlung zu Ozon erhöht wird. Außerdem ermöglicht der Sauerstoffkonzentrator, dass die Menge des erzeugten Ozons unabhängig von äußeren Einflüssen, wie beispielsweise der Raumluftfeuchte, im Wesentlichen konstant bleibt.

Ferner kann dem Luftverdichter eine Einrichtung zur Luftfilterung, insbesondere ein Molekularsieb, nachgeordnet sein, die sauerstofffremde Luftbestandteile, insbesondere Stickstoff, aus der Luft filtert. Auf diese Weise wird der dem Ozonisator zugeführte Sauerstoffanteil weiter erhöht und damit dessen Effizienz gesteigert.

Bei einer bevorzugten Ausführungsform der erfindungsgemäßen Vorrichtung ist ein Vorratsbehälter für Wasser vorgesehen. Auf diese Weise kann die Vorrichtung betrieben werden, ohne dass ein Anschluss an ein Wassernetz notwendig ist. Die Kombination der Reinigungsvorrichturig mit einem Wasserbehälter ist vor allem für den mobilen Einsatz der Vorrichtung zweckmäßig. Für den stationären Einsatz ist ein Wasserbehälter nicht notwendig. Allerdings ist eine Wasserzufuhr, beispielsweise durch eine Wasserzuleitung, für den Betrieb der Vorrichtung im Allgemeinen zweckmäßig.

Vorzugsweise ist die Vorrichtung fahr- oder tragbar, so dass ein mobiler Einsatz der erfindungsgemäßen Vorrichtung einfach möglich ist.

Bei einer weiteren bevorzugten Ausführungsform der erfindungsgemäßen Vorrichtung umfasst die Ventilationseinrichtung einen Lufterhitzer. Dadurch kann die Raumtemperatur des geschlossenen Raumes erhöht werden, so dass die Effizienz des anschließenden Reinigungsvorgangs erhöht wird.

Des Weiteren kann die erfindungsgemäße Vorrichtung zumindest eine Kontrollanzeige, insbesondere Kontrolllampe, aufweisen, die die Ozonkonzentration im geschlossenen Raum anzeigt. Auf diese Weise können anwesende Personen gewarnt werden, wenn die Ozonkonzentration ein gesundheitsschädliches Niveau erreicht hat. Ferner kann der automatisierte Betrieb der Vorrichtung sowie der Erfolg der Ozonzerstörung mittels Wasserdampf kontrolliert werden.

Gemäß einem nebengeordneten Aspekt beruht die Erfindung auf dem Gedanken, ein Verfahren zum Reinigen geschlossener Räume unter Dampferzeugen und/oder Befeuchten von Luft und Ventilieren anzugeben, wobei das Dampferzeugen und/oder Luftbefeuchten und das Ventilieren derartig gesteuert werden, dass die Zeitdauer für das Einleiten des Dampfes und/oder der befeuchteten Luft in den geschlossenen Raum und dessen Einwirkzeit im geschlossenen Raum gesteuert werden können. Dabei wird mit einem Ozonisator Ozon erzeugt, wobei eine Einrichtung zum Ventilieren und/oder eine Steuereinrichtung derart gekoppelt werden, dass die Zeit für das Einleiten des Ozons in den geschlossenen Raum und/oder die Einwirkzeit des Ozons steuerbar ist. Mit dem erfindungsgemäßen Verfahren wird eine besonders effiziente und schonende Reinigung geschlossener Räume erreicht. Weitere Vorteile sind im Zusammenhang mit der Vorrichtung offenbart und gelten gleichermaßen für das erfindungsgemäße Verfahren.

Vorzugsweise wird beim Reinigen die Temperatur auf Raumtemperatur belassen bzw. auf etwa 15 - 35 °C eingestellt. Bei diesen Temperaturen wird eine besonders effiziente Reinigungswirkung erzielt. Eine Reinigung bei Raumtemperatur ermöglicht außerdem eine energieeffiziente Durchführung des Verfahrens.

Vorzugsweise wird die relative Feuchtigkeit in dem zu reinigenden, geschlossenen Raum auf mehr als 50%, insbesondere mehr als 60%, insbesondere mehr als 75%, insbesondere mehr als 80% eingestellt. Durch eine hohe relative Feuchtigkeit wird erreicht, dass die zur Reinigung eingesetzten Ozonmoleküle relativ schnell zerstört werden, so dass bereits nach kurzer Zeit die Ozonkonzentration in dem geschlossenen Raum reduziert wird. Somit kann eine schnelle Reinigung gewährleistet werden. Der gereinigte Raum kann also nach kurzer Zeit wieder betreten werden, ohne dass die Gefahr besteht, dass gesundheitliche Schäden auftreten.

Vorzugsweise wird die Ozonkonzentration im geschlossenen Raum auf mehr als 2 ppm, insbesondere mehr als 20 ppm, insbesondere mehr als 100 ppm, eingestellt. Die Ozonkonzentration in dem geschlossenen Raum wird vorteilhafterweise auf höchstens 300 ppm eingestellt. Auf diese Weise kann die Reinigungsleistung beeinflusst werden. Eine geringe Ozonkonzentration wird bevorzugt zur Reinigung von geschlossenen Räumen eingesetzt, die einen geringen Verschmutzungs- bzw. Kontaminationsgrad aufweisen. Durch die geringe Ozonkonzentration erfolgt die Ozonzerstörung durch den Wasserdampf relativ schnell, so dass der Reinigungsvorgang nach kurzer Zeit beendet ist. Dieser Effekt kann durch die in den geschlossenen Raum gestellte Luftfeuchte beeinflusst werden, wobei der Zeitbedarf für die Reinigung mit Erhöhung der relativen Feuchtigkeit reduziert wird. Für stark kontaminierte geschlossene Räume wird die Ozonkonzentration vorzugsweise auf einem höheren Wert, beispielsweise 20 ppm - 100 ppm, eingestellt.

Bei einer bevorzugten Ausführungsform des erfindungsgemäßen Verfahrens werden die Temperatur, die relative Luftfeuchtigkeit und die Ozonkonzentration beim Reinigen des geschlossenen Raumes derart eingestellt, dass das Reinigen nicht mehr als etwa drei Stunden, insbesondere nicht mehr als eine Stunde beträgt. Durch entsprechende Einstellung der drei wesentlichen Parameter, nämlich Temperatur, relative Feuchtigkeit und Ozonkonzentration, ist es möglich, eine hohe Reinigungsleistung bei geringem Zeitaufwand zu erreichen. Ferner können die Parameter derart eingestellt werden, dass zusätzlich zur hohen Reinigungswirkung und zum geringen Zeitbedarf eine vorteilhafte Energieeffizienz erreicht wird.

Die Erfindung wird im Folgenden anhand von Ausführungsbeispielen unter Bezug auf die beigefügten Zeichnungen näher erläutert. Darin zeigen
- Fig. 1: einen schematischen Aufbau einer Vorrichtung zur Reinigung geschlossener Räume.gemäß einem erfindungsgemäßen Ausführungsbeispiel; und
- Fig. 2: einen schematischen Aufbau einer Vorrichtung zur Reinigung geschlossener Räume gemäß einem weiteren erfindungsgemäßen Ausführungsbeispiel.

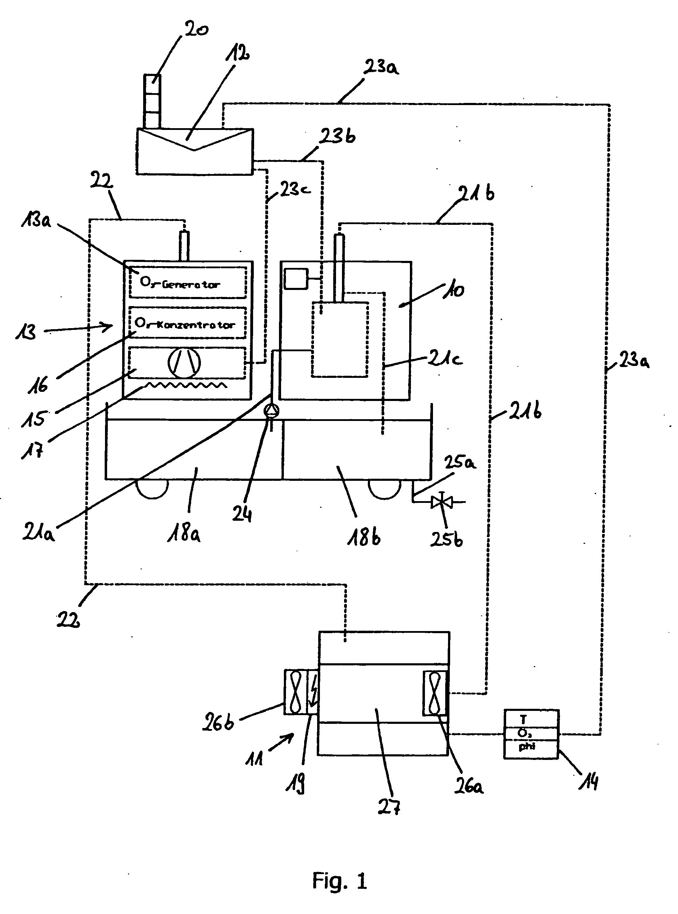

Gemäß Fig. 1 umfasst das erfindungsgemäße Ausführungsbeispiel einer Vorrichtung zur Reinigung geschlossener Räume einen Dampferzeuger 10, eine Ventilationseinrichtung 11, eine Steuereinrichtung 12, einen Ozonisator 13, der mit einem Messwertaufnehmer 14 gekoppelt ist, sowie einen Wassertank 18a und einen Kondensattank 18b.

Der Dampferzeuger 10 weist eine Wasserleitung 21a auf, die eine Pumpe 24 umfasst und den Wassertank 18a mit dem Dampferzeuger 10 verbindet. Ferner ist eine Kondensatleitung 21c vorgesehen, die den Dampferzeuger 10 mit dem Kondensattank 18b verbindet. Der Kondensattank 18b weist außerdem eine Abwasserleitung 25a auf, die ein Abwasserventil 25b umfasst. Der Dampferzeuger 10 ist mit der Ventilationseinrichtung 11 durch eine Dampfleitung 21b verbunden. Anstelle oder zusätzlich zum Dampferzeuger kann ein Luftbefeuchter, beispielsweise ein Hochdruckbefeuchter oder Ultraschallbefeuchter oder ein anderer Befeuchter verwendet werden.

Der Ozonerzeuger 13 umfasst einen Ozonisator 13a, einen Sauerstoffkonzentrator 16, einen Luftverdichter 15 sowie einen Luftfilter 17. Der Luftfilter 17 weist vorzugsweise ein Molekularsieb (nicht dargestellt) auf. Der Ozonerzeuger 13 ist durch eine Ozonleitung 22 mit der Ventilationseinrichtung 11 gekoppelt.

Die Ventilationseinrichtung 11 umfasst zwei. Ventilatoren, einen Dampfventilator 26a und einen Raumventilator 26b, wobei dem Raumventilator 26b in Strömungsrichtung ein Lufterhitzer 19, vorzugsweise ein elektrischer Lufterhitzer 19 bzw. Lufttrockner, vorgeordnet ist. Der Dampfventilator 26a ist innerhalb einer Kammer 27 angeordnet, wohingegen der Raumventilator 26b sowie der Lufttrockner bzw. Lufterhitzer 19 außerhalb der Kammer 27 angeordnet sind. In die Kammer 27 zündet die Ozonleitung 22. Die Dampfleitung 21b ist mit dem Dampfventilator 26 gekoppelt, so dass der Dampf durch den Dampfventilator 26a in die Kammer 27 transportiert wird. Der Raumventilator 26b ist hingegen derart angeordnet, dass das Ozon und/oder der Dampf aus der Kammer 27 in den Raum befördert werden kann.

Ferner ist eine Steuereinrichtung 12 vorgesehen, die eine Kontrollanzeige 20 umfasst. Die Steuereinrichtung 12 weist drei Steuerleitungen 23a, 23b, 23c auf, die eine Verbindung der Steuereinrichtung 12 mit dem Messwertaufnehmer 14 durch die Steuerleitung 23a, mit dem Dampferzeuger 10 durch die Steuerleitung 23b und mit dem Ozonerzeuger 13 durch die Steuerleitung 23c herstellen. Die Steuerleitung 23a ist ferner mit der Ventilationseinrichtung 11 gekoppelt, wobei der Messwertaufnehmer 14 zwischen die Steuereinrichtung 12 und der Ventilationseinrichtung 11 geschaltet ist.

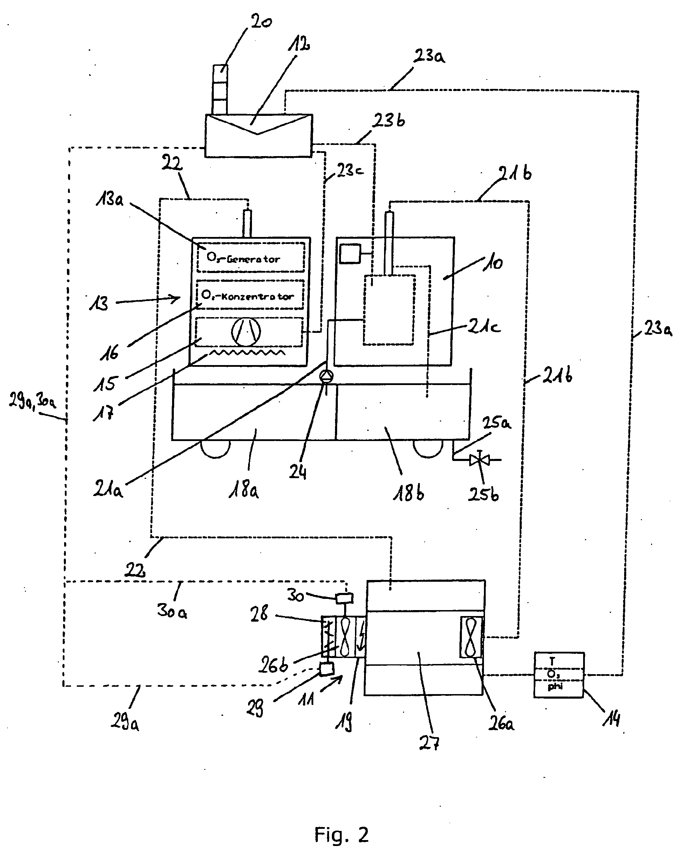

In Fig. 2 ist ein weiteres Ausführungsbeispiel der Reinigungsvorrichtung dargestellt, wobei diese Ausführungsform vorzugsweise für den mobilen Einsatz vorgesehen ist. Der Aufbau der Vorrichtung gemäß Fig. 2 entspricht im Wesentlichen dem Aufbau gemäß Fig. 1, wobei die Ventilationseinrichtung 11 zusätzlich eine Luftklappe 28 mit einem Steuermotor 29 und einem Frequenzumrichter 30 umfasst. Aus Gründen der Übersichtlichkeit wird auf eine Beschreibung der aus Fig. 1 bekannten Bauteile verzichtet, die mit denselben Bezugszeichen versehen sind.

Die Luftklappe 28 ist dem Raumventilator 26b vorgeordnet und reguliert zusammen mit dem Raumventilator 26b die Zuluftmenge zur Kammer 27. Die Luftklappe 28 ist mit dem Steuermotor 29 verbunden, der die Betriebsstellung der Luftklappe 29 reguliert. Der Steuermotor 29 ist durch eine Steuerleitung 29a mit der Steuereinrichtung 12 gekoppelt. Die Steuereinrichtung 12 ist ferner mit dem Frequenzumrichter 30 verbunden, der die Drehzahl des Raumluftventilators 26b abgreift und über die Steuerleitung 30a an die Steuereinrichtung 12 übermittelt, so dass durch die Steuereinrichtung 12 die zugeführte Luftmenge bestimmt werden kann. Auf Grundlage der Drehzahl des Raumluftventilators 26b bzw. der Zuluftmenge erfolgt die Steuerung des Steuermotors 29 bzw. der Luftklappe 28. Bei vollständig geschlossener Luftklappe wird die Vorrichtung im Umluftbetrieb genutzt, d.h. ohne Frischluftzufuhr über den Raumventilator 26b. Die Verwendung des Umluftbetriebs ist besonders bevorzugt, da auf diese. Weise die für die Reinigung notwendige Ozonmenge reduziert werden kann, so dass insgesamt die Effizienz der Vorrichtung und des Reinigungsvorgangs bzw. -verfahrens gesteigert wird.

Die Anwendungsmöglichkeiten für die erfindungsgemäße Vorrichtung sind vielfältig. Beispielsweise kann die Vorrichtung zur Desinfektion von Produktionsprozessen in der Pharmazie, für die Desinfektion von Klimaanlagen oder Klimaanlagensystemen sowie Wärmetauschern eingesetzt werden. Weitere Anwendungsmöglichkeiten finden sich beispielsweise im Bereich der Personenbeförderung, so dass die Vorrichtung besonders geeignet ist zur Reinigung von Bussen, Flugzeugen, Bahnen oder anderen Fahrzeugen. Durch die geruchszerstörende Wirkung des Ozons eignet sich die Vorrichtung insbesondere zur Reinigung von Tiertransporten. Ferner kann eine erfindungsgemäße Vorrichtung zur Reinigung und Sanierung von Bekleidungsstücken wie Schuhen, Helmen oder Schutzanzügen genutzt werden. Weitere Einsatzmöglichkeiten bestehen in der Sanierung von Räumen wie Kellern, Schlachtereien, Küchen oder Hotelzimmern. Insbesondere in stark geruchsbelasteten Räumen können die Vorteile der Reinigung mit Ozon genutzt werden.

Die Vorrichtung kann sowohl stationär als auch mobil betrieben werden. Bevorzugt ist eine mobile Vorrichtung, bei der durch den Wassertank 18a auf einen vorhandenen Wasseranschluss verzichtet werden kann. Ein direkter Anschluss an das Wassernetz ist dennoch möglich. Während des Betriebs der Vorrichtung wird aus dem Wassertank 18a mittels der Pumpe 24 das Wasser durch die Wasserleitung 21a in den Dampferzeuger 10 gepumpt. Im Dampferzeuger 10 wird das Wasser verdampft und durch die Dampfleitung 21b und den Dampfventilator 26a in die Kammer 27 der Ventilationseinrichtung 11 befördert. Im Dampferzeuger 10 anfallendes Kondensat wird durch die Kondensatleitung 21c zum Kondensattank 18b geleitet. Es ist möglich, dass das Kondensat aus dem Kondensattank 18b für die Dampferzeugung im Dampferzeuger 10 wieder verwendet wird. Der Kondensattank 18b kann über die Abwasserleitung 25a durch Betätigung des Abwasserventils 25b entleert werden.

Zur Ozonerzeugung wird Raumluft durch den Luftfilter 17 geleitet, der vorzugsweise als Molekularsieb ausgebildet ist, so dass sauerstofffremde Luftbestandteile, insbesondere Stickstoff, herausgefiltert werden. Die gefilterte Luft wird durch einen Verdichter 15 zu einem Sauerstoffkonzentrator 16 geleitet, der die Sauerstoffkonzentration in der gefilterten Luft erhöht. Die nun mit Sauerstoff angereicherte Luft wird weiter zu einem Ozonisator 13a geleitet, der aus dem zweiwertigen Sauerstoff das dreiwertige Ozon erzeugt. Das Ozon wird durch die Ozonleitung 22 ebenfalls in die Kammer 27 der Ventilationseinrichtung 11 geleitet.

Durch den Raumventilator 26b kann sowohl das Ozon als auch der Dampf aus der Kammer 27 in den Raum geblasen werden. Die zeitliche Abfolge der einzelnen Vorgänge (Dampferzeugung, Dampfeinleitung in den Raum, Ozonerzeugung, Ozoneinleitung in den Raum) wird durch eine Steuereinrichtung 12 geregelt. Die Steuereinrichtung 12 ist dabei so programmiert, dass zum einen zeitliche Vorgaben für die verschiedenen Arbeitsschritte eingehalten werden, und zum anderen die verschiedenen Arbeitsschritte aufgrund der Messwerte des Messwertaufnehmers 14 gesteuert werden. Die Kontrollanzeige 20 umfasst vorzugsweise drei Kontrollleuchten, die die momentane Ozonkonzentration im Raum anzeigen. Dabei wird die momentane Ozonkonzentration im Raum in die Kategorien niedrig, mittel, hoch eingeteilt.

Die Wirksamkeit und Effizienz der Reinigung mit Ozon hängt im Wesentlichen von vier Faktoren ab: Temperatur, Luftfeuchte, Ozonkonzentration im Raum sowie Behandlungsdauer. Vorzugsweise erfolgt die Reinigung bei einer Raumtemperatur von mehr als 25° C und einer Luftfeuchte von mehr als 80 %. Um dies zu erreichen, wird zunächst die Raumtemperatur durch den Lufterhitzer 19 erhöht. Des Weiteren wird durch den erzeugten Dampf die Luftfeuchte auf das erforderliche Niveau eingestellt. Sobald die erforderlichen Temperatur- und Luftfeuchtewerte erreicht sind, wird durch den Ozonerzeuger 13 die Raumluft aufbereitet und das Ozon in den Raum geleitet. Je nach Anforderung an die Reinigungsleistung liegen die Ozonkonzentrationen vorzugsweise im Bereich von 100 bis 300 ppm. Vorzugsweise erfolgt die Befeuchtung mit Dampf isotherm, da es sonst an den Oberflächen im Raum zu einer Abkühlung kommt und somit die Reaktion des Ozons mit den zu behandelnden Oberflächen vermindert wird. Üblicherweise erfolgen die Dampfeinleitung und die Ozoneinleitung abwechselnd und mehrfach hintereinander. Im letzten Schritt des Reinigungsvorgangs erfolgt nochmals die Einleitung des Wasserdampfes in den Raum, so dass das Ozon zerstört wird.

Die vollständig automatisierte Steuerung kann an verschiedene Anwendungsbedürfnisse angepasst werden. Beispielsweise können in der Programmierung verschiedene Raumgrößen und/oder Raumgeometrien hinterlegt sein. Es ist auch denkbar, dass unterschiedliche Reinigungsgrade in der Programmierung berücksichtigt werden. Auf diese Weise kann der Anwender aus verschiedenen Programmen wählen, die sich durch verschiedene Temperaturen, Luftfeuchtewerte, Ozonkonzentrationen und Behandlungszeiten unterscheiden.

### Bezugszeichenliste

- 10: Dampferzeuger
- 11: Ventilationseinrichtung
- 12: Steuereinrichtung
- 13: Ozonerzeuger
- 13a: Ozonisator
- 14: Messwertaufnehmer
- 15: Luftverdichter
- 16: Sauerstoffkonzentrator
- 17: Luftfilter
- 18a: Wassertank
- 18b: Kondensattank
- 19: Lufterhitzer
- 20: Kontrollanzeige
- 21a: Wasserleitung
- 21b: Dampfleitung
- 21c: Kondensatleitung
- 22: Ozonleitung
- 23a, 23b, 23c, 29a, 30a: Steuerleitungen
- 24: Pumpe
- 25a: Abwasserleitung
- 25b: Abwasserventil
- 26a: Dampfventilator
- 26b: Raumventilator
- 27: Kammer
- 28: Luftklappe
- 29: Steuermotor
- 30: Frequenzumrichter

## Patentansprüche

1. Vorrichtung zur Reinigung geschlossener Räume mit einem Dampferzeuger (10) und/oder Befeuchter und einer Ventilationseinrichtung (11), die mit einer Steuereinrichtung (12) derart gekoppelt sind, dass die Zeit der Dampfeinleitung und/oder der befeuchteten Luft in den geschlossenen Raum und die Einwirkzeit des Dampfes und/oder der befeuchteten Luft im geschlossenen Raum steuerbar und ein Feuchtegehalt im Raum von > 50% einstellbar sind, wobei ein Ozonisator (13) vorgesehen ist, der mit der Ventilationseinrichtung (11) und/oder der Steuereinrichtung (12) derart gekoppelt ist, dass die Zeit der Ozoneinleitung in den geschlossenen Raum und/oder die Einwirkzeit des Ozons im geschlossenen Raum steuerbar und ein Ozongehalt im Raum von mindestens 2ppm einstellbar ist, wobei die Steuereinrichtung (12) derart angepasst ist, dass das Ozon und der Dampf und/oder die befeuchtete Luft wechselweise in den Raum einleitbar sind bzw. dort einwirken.

2. Vorrichtung nach Anspruch 1, **dadurch gekennzeichnet, dass** die Steuereinrichtung (12) mit einem Messwertaufnehmer (14), insbesondere zur Messung von Temperatur und/oder Luftfeuchte und/oder Ozonkonzentration, gekoppelt ist.

3. Vorrichtung nach Anspruch 2, **dadurch gekennzeichnet, dass** die Steuereinrichtung (12) derart angepasst ist, dass die Einleit- und/oder Einwirkzeiträume des Ozons und/oder des Dampfs und/oder der befeuchteten Luft in Abhängigkeit der Messwerte des Messwertaufnehmers (14) steuerbar sind.

4. Vorrichtung nach wenigstens einem der Ansprüche 1 bis 3, **dadurch gekennzeichnet, dass** dem Ozonisator (13) ein Luftverdichter (15) vorgeordnet ist.

5. Vorrichtung nach Anspruch 4, **dadurch gekennzeichnet, dass** dem Luftverdichter (15) ein Sauerstoffkonzentrator (16) nachgeordnet ist, der Sauerstoff aus der Umgebungsluft verdichtet und dem Ozonisator (13) zuführt.

6. Vorrichtung nach Anspruch 4 oder 5, **dadurch gekennzeichnet, dass** dem Luftverdichter (15) eine Einrichtung zur Luftfilterung (17), insbesondere ein Molekularsieb, nachgeordnet ist, die sauerstofffremde Luftbestandteile, insbesondere Stickstoff, aus der Luft filtert.

7. Vorrichtung nach wenigstens einem der Ansprüche 1 bis 6, **dadurch gekennzeichnet, dass** ein Vorratsbehälter für Wasser (18a) vorgesehen ist.

8. Vorrichtung nach wenigstens einem der Ansprüche 1 bis 7, **dadurch gekennzeichnet, dass** die Vorrichtung fahr- oder tragbar ist.

9. Vorrichtung nach wenigstens einem der Ansprüche 1 bis 8, **dadurch gekennzeichnet, dass** die Ventilationseinrichtung (11) einen Lufterhitzer (19) umfasst.

10. Vorrichtung nach wenigstens einem der Ansprüche 1 bis 9, **dadurch gekennzeichnet, dass** zumindest eine Kontrollanzeige (20), insbesondere Kontrolllampe, vorgesehen ist, die die Ozonkonzentration im geschlossenen Raum anzeigt.

11. Verfahren zum Reinigen geschlossener Räume unter Dampferzeugen und/oder Befeuchten von Luft und Ventilieren, wobei das Dampferzeugen und/oder Luftbefeuchten und das Ventilieren derartig gesteuert werden, dass die Zeitdauer für das Einleiten des Dampfes und/oder der befeuchteten Luft in den geschlossenen Raum und dessen Einwirkzeit im geschlossenen Raum gesteuert werden können und ein Feuchtegehalt im Raum von > 50% einstellbar ist, wobei mit einem Ozonisator Ozon erzeugt wird, wobei eine Einrichtung zum Ventilieren und/oder eine Steuereinrichtung derart mit dem Ozonisator gekoppelt werden, dass die Zeit für das Einleiten des Ozons in den geschlossenen Raum und/oder die Einwirkzeit des Ozons steuerbar und ein Ozongehalt im Raum von mindestens 2 ppm einstellbar ist, wobei die Steuereinrichtung derart angepasst wird, dass Ozon und Dampf und/oder befeuchtete Luft wechselweise in den Raum eingeleitet werden bzw. dort einwirken.

12. Verfahren nach Anspruch 11, **dadurch gekennzeichnet, dass** die Steuereinrichtung mit einem Messwertaufnehmer, insbesondere zur Messung von Temperatur und/oder Luftfeuchte und/oder Ozonkonzentration gekoppelt wird.

13. Verfahren nach Anspruch 12, **dadurch gekennzeichnet, dass** die Steuereinrichtung derart angepasst wird, dass die Einleit- und/oder Einwirkzeiten des Ozons und/oder des Dampfs und/oder der befeuchteten Luft in Abhängigkeit zu den Messwerten des Messwertaufnehmers gesteuert werden.

## Claims

1. A device for cleaning closed spaces with a steam generator (10) and/or humidifier and/or ventilation mechanism (11), which are coupled to a control mechanism (12) in such a way that the time for introducing steam and/or humidified air into the closed space and the action time of the steam and/or humidified air in the closed space can be controlled and a moisture content of > 50% can be adjusted in the space, an ozoniser (13) being provided, which is coupled to the ventilation mechanism (11) and/or the control mechanism (12) in such a way that the time for introducing ozone into the closed space and/or the action time of the ozone in the closed space can be controlled and an ozone content in the space of at least 2 ppm can be adjusted, wherein the control mechanism (12) is adapted in such a way that the ozone and the steam and/or the humidified air can be introduced alternately into the space and act there.

2. A device according to claim 1, **characterised in that** the control mechanism (12) is coupled to a measuring sensor (14), especially for measuring temperature and/or air humidity and/or ozone concentration.

3. A device according to claim 2, **characterised in that** the control mechanism (12) is adapted in such a way that the introduction and/or action time periods of the ozone and/or the steam and/or the humidified air can be controlled as a function of the measured values of the measuring sensor (14).

4. A device according to at least any one of claims 1 to 3, **characterised in that** an air compressor (15) is arranged upstream of the ozoniser (13).

5. A device according to claim 4, **characterised in that** an oxygen concentrator (16) is arranged downstream of the air compressor (15) and compresses oxygen from the ambient air and supplies it to the ozoniser (13).

6. A device according to claim 4 or claim 5, **characterised in that** a mechanism for air filtration (17), especially a molecular sieve, which filters air components different from oxygen, especially nitrogen, from the air, is arranged downstream of the air compressor (15).

7. A device according to at least any one of claims 1 to 6, **characterised in that** a storage container for water (18a) is provided.

8. A device according to at least any one of claims 1 to 7, **characterised in that** the device can be moved or carried.

9. A device according to at least any one of claims 1 to 8, **characterised in that** the ventilation mechanism (11) comprises an air heater (19).

10. A device according to at least any one of claims 1 to 9, **characterised in that** at least one control display (20), especially a control lamp, is provided, which displays the ozone concentration in the closed space.

11. A method for cleaning closed spaces with steam generation and/or air humidification and ventilation, wherein the steam generation and/or the air humidification and the ventilation are controlled in such a way that the time period for introducing the steam and/or the humidified air into the closed space and the action time thereof in the closed space can be controlled and a moisture content of > 50% can be adjusted in the space, ozone being produced by an ozoniser, wherein a mechanism for ventilation and/or a control mechanism are coupled to the ozoniser in such a way that the time for introducing ozone into the closed space and/or the action time of the ozone can be controlled and an ozone content in the space of at least 2 ppm can be adjusted, the control mechanism being adapted in such a way that ozone and steam and/or humidified air are alternately introduced into the space or act there.

12. A method according to claim 11, **characterised in that** the control mechanism is coupled to a measuring sensor, especially for measuring temperature and/or air humidity and/or ozone concentration.

13. A method according to claim 12, **characterised in that** the control mechanism is adapted in such a way that the introduction and/or action periods of the ozone and/or of the steam and/or the humidified air are controlled as a function of the measured values of the measuring sensor.

## Revendications

1. Dispositif pour nettoyer des espaces fermés comprenant un générateur de vapeur (10) et/ou un humidificateur et un dispositif de ventilation (11), lesquels sont couplés avec un dispositif de commande (12) de telle sorte que le temps de l'introduction de vapeur et/ou de l'air humidifié dans l'espace fermé et le temps d'action de la vapeur et/ou de l'air humidifié dans l'espace fermé peut être contrôlé et une teneur en humidité dans l'espace supérieure à 50 % est réglable, un ozoniseur (13) étant prévu, lequel est couplé avec le dispositif de ventilation (11) et/ou le dispositif de commande (12) de telle sorte que le temps d'introduction de l'ozone dans l'espace fermé et/ou le temps d'action de l'ozone dans l'espace fermé est contrôlable et la teneur en ozone dans l'espace d'au moins 2 ppm est réglable, le dispositif de commande (12) étant adapté de telle sorte que l'ozone et la vapeur et/ou l'air humidifié peuvent être introduits de façon alternative dans l'espace et agir à cet endroit.

2. Dispositif selon la revendication 1, **caractérisé en ce que** le dispositif de commande (12) est couplé avec un enregistreur de valeurs de mesurées (14), en particulier pour la mesure de la température et/ou de l'humidité de l'air et/ou de la concentration d'ozone.

3. Dispositif selon la revendication 2, **caractérisé en ce que** le dispositif de commande (12) est adapté de telle sorte que les périodes d'introduction et/ou d'action de l'ozone et/ou de la vapeur et/ou de l'air humidifié peuvent être contrôlées en fonction des valeurs de mesure de l'enregistreur de valeurs mesurées (14).

4. Dispositif selon au moins l'une des revendications 1 à 3, **caractérisé en ce qu'**un compresseur d'air (15) est disposé en amont de l'ozoniseur (13).

5. Dispositif selon la revendication 4, **caractérisé en ce qu'**un concentrateur d'oxygène (16), qui comprime l'oxygène provenant de l'air ambiant et l'amène à l'ozoniseur (13), est disposé en aval du compresseur d'air (15).

6. Dispositif selon la revendication 4 ou 5, **caractérisé en ce qu'**un dispositif pour le filtrage de l'air (17), en particulier un tamis moléculaire, est disposé en aval du compresseur d'air (15), lequel dispositif élimine par filtrage de l'air des composants de l'air étrangers à l'oxygène, en particulier l'azote.

7. Dispositif selon au moins l'une des revendications 1 à 6, **caractérisé en ce qu'**un conteneur réservoir est prévu pour de l'eau (18a).

8. Dispositif selon au moins l'une des revendications 1 à 7, le dispositif étant **caractérisé en ce qu'**il est mobile ou portable.

9. Dispositif selon au moins l'une des revendications 1 à 8, **caractérisé en ce que** le dispositif de ventilation (11) comprend un réchauffeur d'air (19).

10. Dispositif selon au moins l'une des revendications 1 à 9, **caractérisé en ce qu'**au moins un affichage de contrôle (20), en particulier une lampe témoin, est prévu, lequel indique la concentration d'ozone dans l'espace fermé.

11. Procédé pour nettoyer des espaces fermés avec production de vapeur et/ou humidification d'air et ventilation, la production de vapeur et/ou l'humidification de l'air et la ventilation étant contrôlées de telle sorte que la durée pour l'introduction de la vapeur et/ou de l'air humidifié dans l'espace fermé et son temps d'action dans l'espace fermé peuvent être contrôlés et une teneur en humidité dans l'espace supérieure à 50 % est réglable, de l'ozone étant généré avec un ozoniseur, un dispositif pour la ventilation et/ou un dispositif de commande étant couplés avec l'ozoniseur de telle sorte que le temps pour l'introduction de l'ozone dans l'espace fermé et/ou le temps d'action de l'ozone est contrôlable et une teneur en ozone dans l'espace d'au moins 2 ppm est réglable, le dispositif de commande étant adapté de telle sorte que l'ozone et la vapeur et/ou l'air humidifié sont introduits alternativement dans l'espace et agissent à cet endroit.

12. Procédé selon la revendication 11, **caractérisé en ce que** le dispositif de commande est couplé avec un enregistreur de valeurs mesurées, en particulier pour la mesure de la température et/ou de l'humidité de l'air et/ou de la concentration d'ozone.

13. Procédé selon la revendication 12, **caractérisé en ce que** le dispositif de commande est adapté de telle sorte que les temps d'introduction et/ou d'action de l'ozone et/ou de la vapeur et/ou de l'air humidifié sont commandés en fonction des valeurs mesurées de l'enregistreur de valeurs mesurées.
